# EUROPEAN PATENT SPECIFICATION

(11) **EP 2 645 949 B1**
(45) Date of publication and mention of the grant of the patent: **29.06.2016**
(21) Application number: 11787660.7
(22) Date of filing: 16.11.2011
(51) Int. Cl.: A61B 17/70

(54) **ROD HOLDING DEVICE**
STANGENHALTEVORRICHTUNG
DISPOSITIF DE MAINTIEN DE TIGE

(30) Priority: 03.12.2010 EP 10306348
(43) Date of publication of application: 09.10.2013
(73) Proprietor: ZIMMER SPINE, 33000 Bordeaux (FR)
(72) Inventor: DOUGET, Stéphane, 29390 Scaer (FR); LARROQUE, Gilles, F-64150 Lagor (FR)
(74) Representative: Mays, Julie
(86) International application number: PCT/EP2011/070215
(87) International publication number: WO 2012/072413

(56) References cited:
- EP-A1- 2 052 689
- EP-A1- 2 105 101
- US-A- 5 010 879
- US-A1- 2002 035 366
- US-A1- 2008 167 688
- US-A1- 2009 264 931

## Description

### TECHNICAL FIELD

The systems and devices, in this disclosure relate to a rod holding device for binding a rod to an osseous structure, in particular a spinal structure.

One field of application for the embodiments in this disclosure is holding bones in a relative position, for example to aid in the healing of breaks or the positioning of bones in the treatment of spinal deformities or spinal degenerative diseases or trauma diseases, or otherwise to correct abnormal curvatures of the spine. Other bone deficiencies and abnormalities may also benefit from embodiments in this disclosure.

### BACKGROUND

The spine is formed of superposed vertebrae, normally aligned along a vertebral axis, from the lumbar vertebrae to the cervical vertebrae, each having a posterior wall from which projects a spinous process and two lateral edges from the walls of which there project ribs and/or transverse processes and/or lamina. If the spine of a person has abnormal curvature, the vertebrae are typically inclined relative to one another and relative to said vertebral axis.

In order to straighten the vertebral column as a remedy for this situation, the lateral edges of the vertebrae on the concave side can be moved away from one another and supported at distances from one another substantially equivalent to the distances between the lateral edges on the other side. Devices known in the art for holding the vertebrae relative to one another include rods that are held by supports attached to the vertebrae, for example using screws, hooks, or flexible ligatures.

One such device is described in European patent application publication EP 2052689 A1. This rod holding device comprises a holding body having a receiving portion for receiving the rod and an engagement portion, a closure member for engagement with the engagement portion of the holding body to secure the rod within said receiving portion, and an anchor member for anchoring the holding body to the osseous structure.

In the rod holding device described in EP 2052689 A1, to ensure a reliable connection, the receiving portion of the holding body has to be calibrated specifically to the gauge of the rod used. Since, depending on the specific intervention and patient, a variety of different rod gauges may be required, blocking bodies adapted to each different rod gauge will have to be produced. US 5010879 describes a rod fixed to hook-line engagement members. Wedge-like members are used to fix the hook members with the rod. EP 2105101A describes a bone anchoring device using a sleeve around the rod in the receiving part of the device.

It has also been disclosed, for instance in Patent Application Publication US 2004/0254574 A1, to receive the rod in an orifice in a ball-shaped intermediate element received itself within the holding body in order to form a ball-and-socket articulation allowing the rod to be swivelled within a certain angle. However, in some cases, such angular movement may not be required, or even appropriate. For example, when the rod and rod holding device are used to stabilize a spine, such a ball-and-socket connection may not provide sufficient stability against forces tending to bend the stabilized spine. Furthermore, size restraints, combined with the ball shape of the intermediate element, limit the length of rod that can be clamped, and therefore the clamping force.

### SUMMARY

An exemplary use of the systems and devices hereby disclosed is that of providing a rod holding device for binding a rod to an osseous structure that can be reliably adapted to a variety of rods of different gauges and diameters, without impairing the strength of their connection.

Accordingly, in at least one illustrative embodiment, a rod holding device comprises a holding body having an engagement portion and a receiving portion, an anchor member for anchoring the holding body to the osseous structure, a closure member for engagement with the engagement portion of the holding body, and a sleeve member with an opening for receiving the rod and a non-spherical outer contact surface for engaging a complementary inner contact surface of said receiving portion of the holding body, wherein the sleeve member is configured to be locked in said receiving portion of the holding body by engagement of the closure member with the engagement portion of the holding body, and to secure the rod within said opening.

Consequently, through the sleeve member, a single type of holding body can be adapted to rods of a variety of different sizes. The engagement of the non-spherical contact surface with a complementary inner contact surface of the receiving portion of the holding body safely secures the sleeve member against unwanted rotation, which can be particularly useful when securing the osseous structure against bending forces.

Advantageously, the sleeve member may be elongated, and the opening for receiving the rod longitudinally oriented. This further secures the sleeve member against unwanted rotation around axes perpendicular to the longitudinal axis of the rod.

Advantageously, said sleeve member may be deformable perpendicularly to the longitudinal axis of the rod. This allows the rod to be secured within the opening in the sleeve member by a clamping pressure between the closure member and the holding body, which is transmitted, through the deformation of the sleeve member, to an outer surface of the rod. Fixing the rod in the sleeve member and the sleeve member to the blocking member can thus be done in a single operation. The longitudinal orientation of the opening in the elongated sleeve member allows a wider distribution of the clamping pressure along its length, and thus higher total clamping forces.

Alternatively, said sleeve member may comprise a pressure element moveable substantially perpendicularly to the longitudinal axis of the rod. For instance, said pressure element may comprise a screw thread engaging a complementary screw thread of the sleeve member. While this will normally require separate operations for fixing the rod in the sleeve member and the sleeve member to the holding body, for this same reason it will allow an adjustment of the longitudinal position of the rod without having to unlock the sleeve member.

It must be noted that, within the present disclosure, the words "alternative" and "alternatively" should not be understood in an exclusionary manner unless explicitly required. Features of various alternative embodiments may thus be combined according to circumstances as the skilled person may find adequate.

The sleeve member presents, at opposite longitudinal ends, axial stops opposed to corresponding outer surfaces of the holding body, so as to limit the axial movement of the sleeve member within the holding body even before the closure member is closed, and thus facilitate handling the assembly.

Advantageously, the sleeve member may be configured to be form-locked in said receiving portion of the holding body, thus ensuring a particularly reliable connection between the sleeve member and the holding body. However, the sleeve member may instead be configured to be frictionally locked in said receiving portion of the holding body.

Various alternatives are also available for the anchor member:
In a first alternative, the anchor member comprises a bone screw, in particular a pedicle screw for fixation to a vertebral pedicle. This alternative offers a particularly secure, rigid anchor to the osseous structure. In particular, for ease of operation, the bone screw may be pivotably connected to the holding body, allowing the user to adapt its orientation.

In a second alternative, the anchor member comprises a flexible elongate member. This alternative offers thus the possibility of a resilient connection to the osseous structure that may in particular be used even in damaged and/or brittle bones that may not be suitable for other anchoring methods. In particular, the flexible elongage member is configured to be frictionally held between the sleeve member and the receiving portion of the holding body by engagement of the closure member with the engagement portion of the holding body. This will thus allow locking the flexible elongate body and the sleeve member to the holding body in a single operation.

In a third alternative, the anchor member comprises a hook, in particular a hook solid with the holding body. This will thus allow the holding body to be quickly and easily anchored to the osseous structure.

Advantageously, the closure member may comprise a screw thread and the engagement portion of the holding body a complementary screw thread, allowing a secure engagement and eventually a strong clamping force between the closure member and the holding body.

Advantageously, the closure member may be hinged to the holding body, allowing the closure member and holding body to be handled as a single part in the operating theatre, and thus simplifying operations.

Also described but not claimed is a method for tying a rod to an osseous structure, and namely comprising the steps of inserting a rod through an opening in a sleeve member, anchoring a holding body to the osseous structure, receiving said sleeve member in a receiving portion of the holding body, locking said sleeve member in said receiving portion of the holding body by engagement of the closure member with an engagement portion of the holding body, and securing the rod within the opening in the sleeve member. It must be noted that these steps may not necessarily be performed in the listed order. In particular, although it will usually be advantageous to preassemble the rod and the sleeve member before the operation, the step of inserting the rod through the opening in the sleeve member may instead be performed after having already anchored the holding body to the osseous structure.

The above summary of some example embodiments is not intended to describe each disclosed embodiment or every implementation of the exemplary embodiments hereby disclosed. In particular, selected features of any illustrative embodiment within this specification may be incorporated into an additional embodiment unless clearly stated to the contrary.

### BRIEF DESCRIPTION OF THE DRAWINGS

The present disclosure may be more completely understood in consideration of the following detailed description of various embodiments in connection with the accompanying drawings, in which :
- FIG. 1 shows a perspective view of a rod-holding device according to a first embodiment;
- FIG. 2 shows a perspective view of a sleeve member of the rod-holding device of FIG. 1;
- FIG. 3 shows a longitudinal section of the rod-holding device of FIG. 1 along plane III-III;
- FIG. 4 shows a cross-section of the rod-holding device of FIG. 1 along plane IV-IV;
- FIG. 5 shows a perspective view of the rod-holding device of FIG. 1, partially assembled;
- FIG. 6 shows a perspective view of a rod-holding device according to a second embodiment;
- FIG. 7 shows a perspective view of a sleeve member of the rod-holding device of FIG. 6;
- FIG. 8 shows a longitudinal section of the rod-holding device of FIG. 6 along line VIII-VIII;
- FIG. 9 shows a transversal section of the rod-holding device of FIG. 6 along line IX-IX;
- FIG. 10 shows a perspective view of the rod-holding device of FIG. 5, partially assembled;
- FIG. 11 shows a longitudinal section of the rod-holding device of FIG. 6, being used to link two different-gauge rods;
- FIGS. 12 and 13 show front cutaway views of two further embodiments.

While the embodiments of this disclosure are amenable to various modifications and alternative forms, specifics thereof have been shown by way of example in the drawings and will be described in detail. It should be understood, however, that the intention is not to limit aspects of the invention to the particular embodiments described. On the contrary, the intention is to cover all modifications, equivalents, and alternatives falling within the scope of the invention.

### DETAILED DESCRIPTION

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

A bone fixing system comprising a plurality of rod holding devices anchored to underlying structures and linked to each other through one or several rods may be installed in a patient to hold or fix one structure in a selected relation with one or more other structures. As used herein, the term structure may refer to bones, portions of bones, or bone implants, as well as rods, elongated members, plates, or other implanted man-made devices. Among other methods, a rod holding device as described herein may be installed using a minimally invasive surgery (MIS) procedure.

Rod holding devices and other components of bone fixing systems in accordance with the disclosure may be made of materials including, but not limited to, titanium, titanium alloys, stainless steel, ceramics, and/or polymers. Some components of a bone fixing system may be autoclaved and/or chemically sterilized. Components that may not be autoclaved and/or chemically sterilized may be made of sterile materials. Components made of sterile materials can be used with other sterile components during assembly of a bone fixing system.

Embodiments of rod holding devices disclosed herein are useful in repairing broken bones, correcting curvatures of the spine and for other surgical procedures that hold structures (e.g., bones) in a fixed relative position. Embodiments of the bone fixing system and method of use disclosed herein can be particularly useful for minimally invasive surgery (MIS) procedures, which can reduce trauma to soft tissue due to the relatively small incision made in a patient. For example, a surgical procedure may be performed through a 2 cm to 4 cm incision formed in the skin of the patient. Dilators, a targeting needle, and/or a tissue wedge may be used to provide access to structures without the need to form a larger incision with a scalpel through muscle and other tissue. A minimally invasive surgery (MIS) procedure may reduce an amount of postoperative pain felt by a patient as compared to invasive procedures. A minimally invasive procedure may also reduce recovery time for the patient as compared to invasive procedures. In some embodiments, the natural flexibility of skin and soft tissue may be used to limit the length and/or depth of an incision or incisions needed during the procedure. Minimally invasive procedures may provide limited direct visibility in vivo.

Bone fixing systems may be used to correct problems due to spinal injury, deformity, or disease. For example, various embodiments of a bone fixing system may be used from the C1 vertebra to the sacrum to correct spinal problems. For example, a bone fixing system may be implanted posterior to the spine to maintain distraction between adjacent vertebral bodies in a lumbar portion of the spine. Various embodiments of a bone fixing system may be used to correct orthopedic deficiencies. Embodiments of the disclosure may be useful for holding tendons, bones, or muscles during the healing process and may be implanted using MIS procedures and thus it is in this context that embodiments of the disclosure may be described. It will be appreciated, however, that embodiments of the systems, devices, and methods of the present disclosure may be applicable for stabilizing other areas of the body.

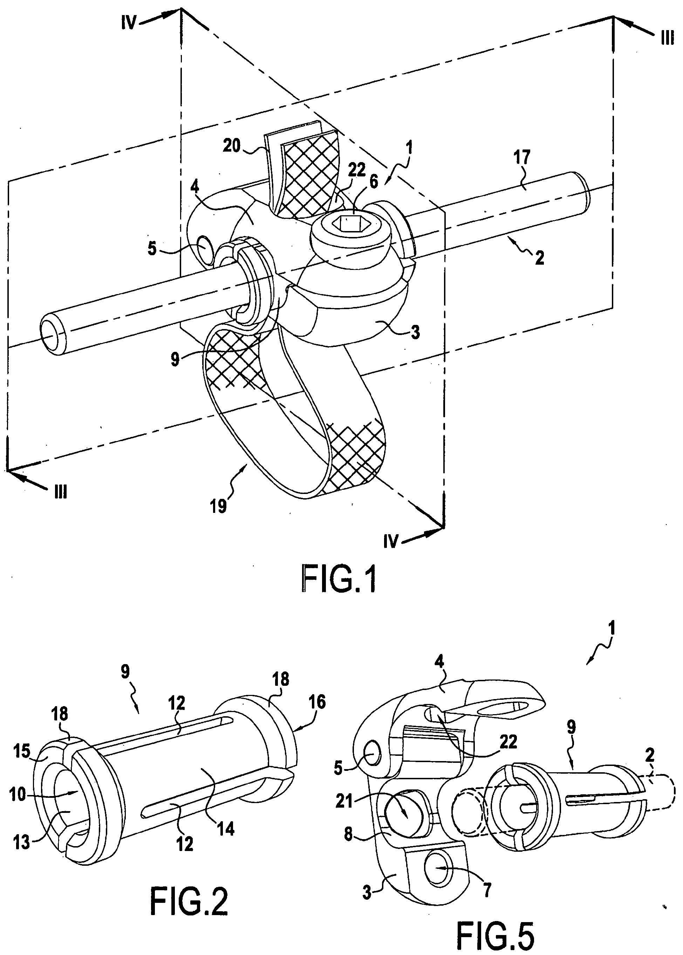

A rod-holding device 1 according to one embodiment is illustrated in FIGS. 1-5. This rod-holding device 1 is intended to bind the rod 2 to an underlying osseous structure (not shown), such as, in particular, vertebrae. The rod 2 can thus be tied to, for example, a spine, for reinforcing, supporting or straightening purposes.

The rod-holding device 1 of this first embodiment is in the form of a clamp comprising a lower holding body 3 and an upper closure member 4, linked to the lower holding body 3 over a hinge 5. The upper closure member 4 also comprises a screw 6 which can be threaded into a complementary screw-threaded orifice 7 forming an engagement portion of the lower holding body 3. The lower holding body 3 and upper closure member 4 can thus be clamped against each other by tightening the screw 6 within the orifice 7.

The lower holding body 3 also comprises a recess 8 forming a receiving portion for an elongated elastic sleeve member 9. This elastic sleeve member 9, separately illustrated in FIG. 2, has a longitudinal orifice 10 for receiving the rod 2, longitudinal slits 12 arranged around the central axis X of the elastic sleeve member 9, extending between an inner surface 13 of the longitudinal orifice 9 and a cylindrical outer surface 14 of the sleeve member, and alternatively starting from each end 15, 16 of the sleeve member 9. As the material of the sleeve member 9 is elastic, a radial pressure on the inner surface 13 will expand the slits 12 and the diameter of the outer surface 14. Inserting a rod 2, with an interference fit, into the central orifice 10 will therefore slightly expand the sleeve member 9, which will exert a pressure on the outer surface 17 of the rod 2. This pressure will cause friction between the outer surface 17 of the rod 2 and the inner surface 13 of the sleeve member 9.

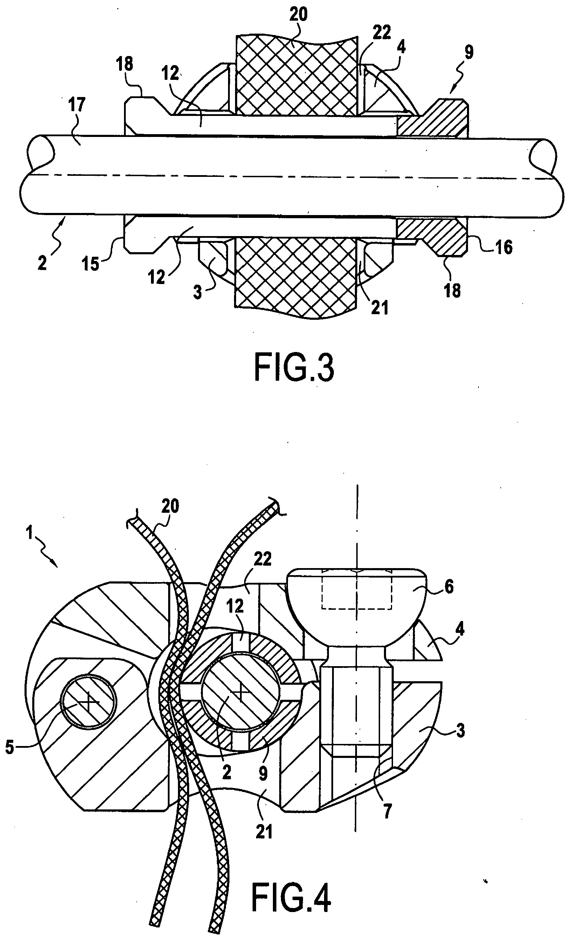

The elastic sleeve member 9 also presents a radial lip 18 at each end 15, 16, with a distance between the lips 18 that is at least equal to the width of the lower holding body 3 and the upper closure member 4, thus forming axial stops opposed to corresponding surfaces of the lower holding body 3 and upper closure member 4. When the elastic sleeve member 9, with the rod 2 within its longitudinal orifice 10, is received into the recess 8 of the lower holding body 3, its longitudinal motion with respect to the lower holding body 3 will thus be limited, as illustrated in FIG. 3. When the screw 6 is tightened to clamp the lower holding body 3 and the upper closure member 4 around the sleeve member 9 and the rod 2, the elastic sleeve member 9 will be frictionally held between the lower holding body 3 and the upper closure member 4, while transmitting the radial clamping pressure of the lower holding body 3 and the upper closure member 4 on its outer surface 13 to the outer surface 17 of the rod 2 so as to, in turn, secure (for example, frictionally holding or clamping) the rod 2 within the orifice 10. Its angular motion around axes orthogonal to the longitudinal axis is also effectively restrained by the shapes of the rod 2, sleeve member 9, lower holding body 3 and upper closure member 4.

To anchor the lower holding body 3 to the underlying osseous structure, the rod-holding device 1 of this first embodiment also comprises an anchor member in the form of a flexible ligature formed by a loop 19 in a flexible band 20. Each one of the lower holding body 3 and the upper closure member 4 present an opening 21, 22 for the flexible band, which is received between the sleeve member 9 and the recess 8 in the lower holding body 3, as illustrated in particular in FIG. 4. When the screw 6 is tightened, the pressure between the recess 8 and the sleeve member 9 will thus also frictionally hold the loop 19, securing in this manner the connection of the rod-holding device 1 to the underlying osseous structure.

In use, elastic sleeve members 9 of appropriate dimensions can be used to adapt rods 2 of various gauges to a single type of clamp. Each elastic sleeve member 9 could be fit to its rod 2 in advance to the operation, so as prevent confusion during the surgery itself. During the surgery, the flexible band is looped around the osseous structure to which the rod-holding device 1 is to be tied, and its two ends inserted through the openings 21, 22 in the lower holding body 3 and upper closure member 4 of the open clamp. The elastic sleeve member 9, with the rod 2, is then received in the recess 8 of the lower holding body 3 of the open clamp, as shown in Fig. 5. The clamp is then closed, and the screw 6 tightened, while the band 20 is held under tension, to clamp the lower holding body 3 and upper closure member 4 around the flexible band 20 and the elastic sleeve member 9, so as to frictionally hold the loop 19, the elastic sleeve member 9, and the rod 2. To subsequently move or adjust any one of them, the screw 6 will then have to be loosened, releasing both the rod 2 and the loop 19.

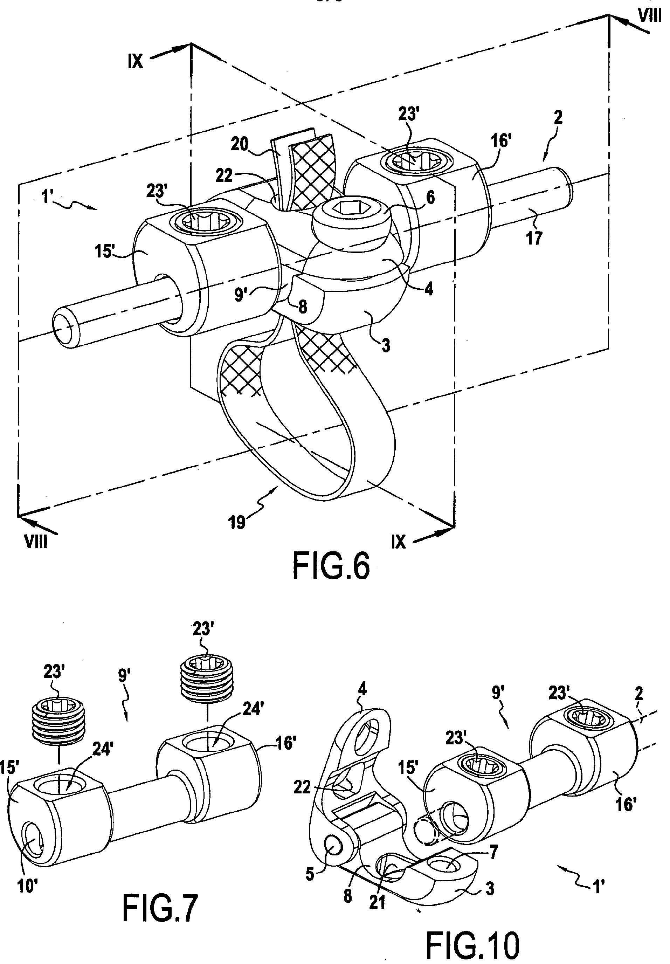

A rod-holding device 1' according to a second embodiment is illustrated in FIGS. 6-11. This rod-holding device 1' comprises a substantially identical flexible band loop 19 and also a substantially identical clamp with lower holding body 3 and upper closure member 4. The parts in this second embodiment therefore receive the same reference numbers as substantially equivalent parts of the first embodiment. However, the rod-holding device 1' of this second embodiment differs from the first embodiment in that the elongated sleeve member 9' is substantially rigid and comprises two pressure elements in the form of lockscrews 23' threaded in radial screw-threaded orifices 24', one at each end 15', 16' of the sleeve member 9'. In this second embodiment, the rod 2 is received with radial play within the longitudinal orifice 10' of the sleeve member 9', and frictionally held by the lockscrews 23' when they are tightened, radially entering the central orifice 10' through the radial orifices 24' and pressing against the outer surface 17 of the rod 2. The rod-holding device 1' of this second embodiment is used in a similar manner as that of the first embodiment, as shown in FIG. 10. As in the first embodiment, the sleeve member 9' can be selected to adapt the rod-holding device 1' to a rod 2 of a particular gauge. However, the use of this rod-holding device 1' of this second embodiment differs from that of the first embodiment in that the rod 2 is held within the sleeve member 8' by the lockscrews 23', independently of the clamping force of the lower holding body 3 and upper closure member 4 on the sleeve member 9' and flexible band 20. The position of the rod 2 may thus be adjusted by loosening the lockscrews 23' without loosening the clamp or the loop 20. The radial screw-threaded orifices 24' are formed in widened sections of the elongated sleeve member 9', which, like the radial lips of the first embodiment, form axial stops limiting the axial movement of the sleeve member 9' within the rod-holding device 1'.

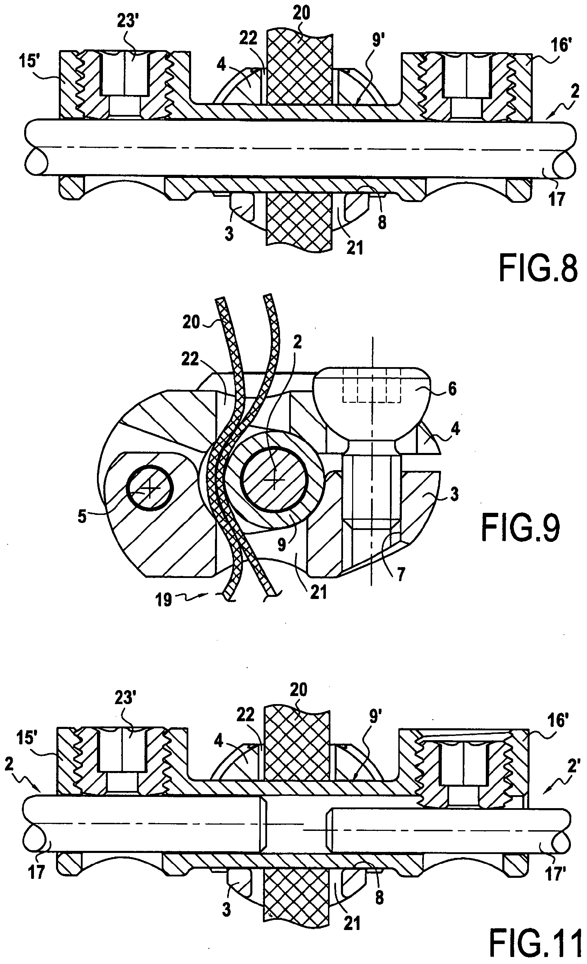

Moreover, as illustrated in FIG. 11, the rod-holding device 1' of this second embodiment can also be used as a rod-to-rod connector for linking two rods 2,2' of different diameters, for instance at the junction between the cervical and thoracic spine. In this use, each lockscrew 23' secures one of the rods 2,2' within the orifice 10' by clamping against its respective outer surface 17, 17'.

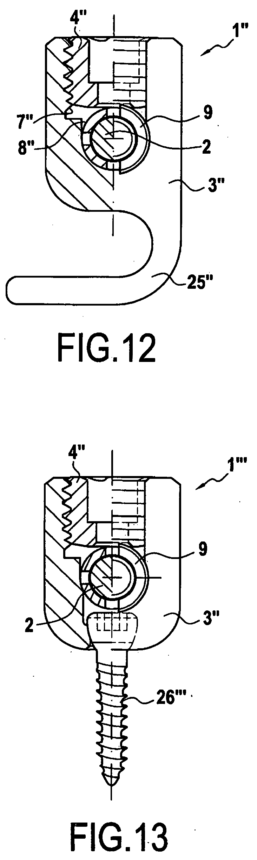

While in the first two embodiments the sleeve member is held within a hinged clamp and the anchor member comprises a flexible elongate member, other embodiments can also be applied to rod-holding devices with different clamping and anchoring means. For instance, FIG. 12 shows a rod-holding device 1" according to a third embodiment, wherein the holding body 3" is in the form of a tulip head with a receiving portion 8" in the form of a deep transversal recess, an upper portion of the side walls of this transversal recess being screw-threaded so as to form an engagement portion 7" for engaging a closure member 4" in the form of a lockscrew. In this third embodiment, the holding body 3" extends into a hook 25" forming an anchor member for anchoring the holding body to the underlying osseous structure. The sleeve member 9 is essentially equivalent to that of the first embodiment. As for the first embodiment, in use, the elastic sleeve member 9 could be fit to its rod 2 in advance to the operation. During the surgery, the holding body 3" is anchored to an underlying osseous structure with the hook 25", and the elastic sleeve member 9, with the rod 2, is then received .in the recess 8". The lockscrew 4" is then threaded to the engagement portion 7" of the holding body 3" and tightened against the elastic sleeve member 9 so as to frictionally lock the sleeve member 9, as well as the rod 2 within.

FIG. 13 shows a rod-holding device 1"' according to a fourth embodiment, wherein the holding body 3", closure member 4", and the elastic sleeve member 9 are essentially equivalent to those of the third embodiment. In this fourth embodiment, however, the anchor member is formed by a bone screw 26"' pivotally connected to the holding body 3". In use, during the surgery, the holding body 3" is anchored to the underlying osseous structure by screwing the bone screw 26" into the bone. Otherwise, it is handled similarly to the third embodiment.

Due to changes in anatomical features, different sized rods may be needed for surgical procedures for different parts of the spine. For example, a 3.5 mm rod may be needed for a cervical spine procedure and 6.0 mm rod may be needed for a lumbar spine procedure. The various hereby disclosed embodiments allow a surgeon to utilize a single rod-holding device and instrument set with rods of variously-sized diameters. This reduces the overall inventory of rod-holding devices and instrumentation needed. In some embodiments, the surgeon has the ability to select from several sleeve size options to fit over the rod and engage the holding body.

Those skilled in the art will recognize that the systems and devices hereby disclosed may be manifested in a variety of forms other than the specific embodiments described and contemplated herein. Accordingly, departure in form and detail may be made without departing from the scope of the systems and devices hereby disclosed as described in the appended claims.

## Claims

1. A rod holding device (1,1',1",1"') for binding a rod (2) to an osseous structure, comprising:
a holding body (3,3") having an engagement portion (7,7") and a receiving portion (8,8");
an anchor member for anchoring the holding body (3,3") to the osseous structure;
a closure member (4,4") for engagement with the engagement portion (7,7") of the holding body (3,3"); and
a sleeve member (9,9') with an opening (10,10') for receiving the rod (2) and a non-spherical outer contact surface for engaging a complementary inner contact surface of said receiving portion (8,8") of the holding body (3,3");
wherein the sleeve member (9,9') is configured to be locked in said receiving portion (8,8") of the holding body (3,3") by engagement of the closure member (4,4") with the engagement portion (7,7") of the holding body (3,3"), and to secure the rod (2) within said opening (10,10') **characterized in that** said sleeve member (9,9') presents, at opposite longitudinal ends, axial stops opposed to corresponding outer surfaces of the holding body (3,3").

2. The rod holding device (1,1',1",1"') of claim 1, wherein the sleeve member (9,9') is elongated, and the opening (10,10') for receiving the rod (2) is longitudinally oriented.

3. The rod holding device (1,1",1"') according to any one of claims 1 or 2, wherein said sleeve member (9) is deformable perpendicularly to a longitudinal axis of the rod (2).

4. The rod holding device (1') according to any one of claims 1 or 2, wherein said sleeve member (9') comprises a pressure element (23') moveable substantially perpendicularly to a longitudinal axis of the rod (2).

5. The rod holding device (1') of claim 4, wherein said pressure element (23') comprises a screw thread engaging a complementary screw thread of the sleeve member (9').

6. The rod holding device (1,1',1",1"') of any one of the previous claims,
wherein the sleeve member (9,9') is configured to be form-locked in said receiving portion (8,8") of the holding body (3,3").

7. The rod holding device (1,1',1",1"') of any one of claims 1 to 5, wherein the sleeve member (9,9') is configured to be frictionally locked in said receiving portion (8,8") of the holding body (3,3").

8. The rod holding device (1"') of any one of the previous claims, wherein the anchor member comprises a bone screw (26"').

9. The rod holding device (1"') of claim 8, wherein said bone screw (26"') is pivotably connected to the holding body (3").

10. The rod holding device (1,1') of any one of the previous claims, wherein the anchor member comprises a flexible elongate member (19).

11. The rod holding device (1,1') of claim 10, wherein said flexible elongate member (19) is configured to be frictionally held between the sleeve member (9,9') and the receiving portion (8,8') of the holding body (3) by engagement of the closure member (4) with the engagement portion (7,7") of the holding body (3).

12. The rod holding device (1") of any one of the previous claims, wherein said anchor member comprises a hook (25").

13. The rod holding device (1") of claim 12, wherein the hook (25") is solid with the holding body (3").

14. The rod holding device (1,1',1",1"') of any one of the previous claims,
wherein the closure member (4,4") comprises a screw thread and the engagement portion (7,7") of the holding body (3,3") comprises a complementary screw thread.

15. The rod holding device (1,1') of any one of the previous claims, wherein the closure member (4) is hinged to the holding body (3).

## Patentansprüche

1. Stabhaltevorrichtung (1,1',1",1"') zur Befestigung eines Stabs (2) an eine Knochenstruktur, aufweisend:
einen Haltekörper (3,3") mit einem Eingriffsabschnitt (7,7") und einen Aufnahmeabschnitt (8,8"),
ein Ankerelement zur Verankerung des Haltekörpers (3,3") an der Knochenstruktur, ein Verschlusselement (4,4") zum Eingriff mit dem Eingriffsabschnitt (7,7") des Haltekörpers (3,3") und
ein Hülsenelement (9,9') mit einer Öffnung (10,10'), um den Stab (2) aufzunehmen, und einer nicht-sphärischen, äußeren Berührungsfläche zum Eingreifen in eine komplementäre, innere Berührungsfläche dieses Aufnahmeabschnitts (8,8") des Haltekörpers (3,3");
wobei das Hülsenelement (9,9') gestaltet ist, um in diesem Aufnahmeabschnitt (8,8") des Haltekörpers (3,3") durch Eingriff des Verschlusselements (4,4") mit dem Eingriffsabschnitt (7,7") des Haltekörpers (3,3") verriegelt zu werden und um den Stab (2) innerhalb dieser Öffnung (10,10') zu sichern, **dadurch gekennzeichnet, dass** dieses Hülsenelement (9,9') an gegenüberliegenden Längsenden axiale Anschläge aufweist, die entsprechenden Außenoberflächen des Haltkörpers (3,3") entgegenstehen.

2. Stabhaltevorrichtung (1,1',1",1"') nach Anspruch 1, wobei das Hülsenelement (9,9') langgestreckt ist und die Öffnung (10,10') zur Aufnahme des Stabs (2) in Längsrichtung orientiert ist.

3. Stabhaltevorrichtung (1,1",1"') nach einem der Ansprüche 1 oder 2, wobei dieses Hülsenelement (9) senkrecht zu einer Längsachse des Stabs (2) verformbar ist.

4. Stabhaltevorrichtung (1') nach einem der Ansprüche 1 oder 2, wobei dieses Hülsenelement (9') ein Druckelement (23') aufweist, dass im Wesentlichen senkrecht zu einer Längsachse des Stabs (2) bewegbar ist.

5. Stabhaltevorrichtung (1') nach Anspruch 4, wobei dieses Druckelement (23') ein Schraubengewinde aufweist, das mit einem komplementären Schraubengewinde des Hülsenelements (9') eingreift.

6. Stabhaltevorrichtung (1,1',1",1"') nach einem der vorgehenden Ansprüche, wobei das Hülsenelement (9,9') gestaltet ist, um formschlüssig in diesem Aufnahmeabschnitt (8,8") des Haltekörpers (3,3") angebracht zu werden.

7. Stabhaltevorrichtung (1,1',1",1"') nach einem der Ansprüche 1 bis 5, wobei das Hülsenelement (9,9') gestaltet ist, um reibschlüssig in diesem Aufnahmeabschnitt (8,8") des Haltekörpers (3,3") angebracht zu werden.

8. Stabhaltevorrichtung (1"') nach einem der vorhergehenden Ansprüche, wobei das Ankerelement eine Knochenschraube (26"') aufweist.

9. Stabhaltevorrichtung (1"') nach Anspruch 8, wobei diese Knochenschraube (26"') schwenkbar am Haltekörper (3") angeschlossen ist.

10. Stabhaltevorrichtung (1,1') nach einem der vorhergehenden Ansprüche, wobei das Ankerelement ein flexibles, längliches Element (19) aufweist.

11. Stabhaltevorrichtung (1,1') nach Anspruch 10, wobei dieses flexible, längliche Element (19) gestaltet ist, um reibschlüssig zwischen dem Hülsenelement (9,9') und dem Aufnahmeabschnitt (8,8') des Haltekörpers (3) durch Eingriff des Verschlusselements (4) mit dem Eingriffsabschnitt (7,7") des Haltekörpers (3) gehalten zu werden.

12. Stabhaltevorrichtung (1") nach einem der vorhergehenden Ansprüche, wobei das Ankerelement einen Haken (25") aufweist.

13. Stabhaltevorrichtung (1") nach Anspruch 12, wobei der Haken (25") mit dem Haltekörper (3") fest verbunden ist.

14. Stabhaltevorrichtung (1,1',1",1"') nach einem der vorgehenden Ansprüche, wobei das Verschlusselement (4,4") ein Schraubengewinde aufweist und der Eingriffsabschnitt (7,7") des Haltekörpers (3,3") ein komplementäres Schraubengewinde aufweist.

15. Stabhaltevorrichtung (1,1') nach einem der vorhergehenden Ansprüche, wobei das Verschlusselement (4) am Haltekörper (3) angelenkt ist.

## Revendications

1. Un dispositif de maintien d'une tige (1,1',1",1"') permettant de fixer une tige (2) sur une structure osseuse, comprenant :
un corps de maintien (3,3") avec une partie d'engagement (7,7") et une partie réceptrice (8,8") ;
un élément d'ancrage pour ancrer le corps de maintien (3,3") sur la structure osseuse ; un élément de fermeture (4,4") pour l'engagement avec la partie d'engagement (7,7") du corps de maintien (3,3") ; et
un élément de manchon (9,9') avec une ouverture (10,10') pour la réception de la tige (2), et une surface de contact extérieure non sphérique pour engager une surface de contact intérieure complémentaire de ladite partie réceptrice (8,8") du corps de maintien (3,3") ;
l'élément de manchon (9,9') étant configuré pour être verrouillé dans ladite partie réceptrice (8,8") du corps de maintien (3,3") par l'engagement de l'élément de fermeture (4,4") avec la partie d'engagement (7,7") du corps de maintien (3,3"), et fixer la tige (2) dans ladite ouverture (10,10'),
**caractérisé en ce que** ledit élément de manchon (9,9') présente, à des extrémités longitudinales opposées, des butées axiales opposées aux surfaces extérieures correspondantes du corps de maintien (3,3").

2. Le dispositif de maintien d'une tige (1,1',1",1"') selon la revendication 1, l'élément de manchon (9,9') étant allongé, et l'ouverture (10,10') de réception de la tige (2) étant orientée longitudinalement.

3. Le dispositif de maintien d'une tige (1,1",1"') selon une quelconque des revendications 1 ou 2, ledit élément de manchon (9) étant déformable perpendiculairement à un axe longitudinal de la tige (2).

4. Le dispositif de maintien d'une tige (1') selon une quelconque des revendications 1 ou 2, ledit élément de manchon (9') comprenant un élément de pression (23') pouvant être déplacé dans une direction substantiellement perpendiculaire à l'axe longitudinal de la tige (2).

5. Le dispositif de maintien d'une tige (1') selon la revendication 4, ledit élément de pression (23') comprenant un filet engageant un filet de vis complémentaire de l'élément de manchon (9').

6. Le dispositif de maintien d'une tige (1,1',1",1"') selon une quelconque des revendications précédentes, ledit élément de manchon (9,9') étant configuré pour être maintenu par engagement positif dans ladite partie réceptrice (8,8") du corps de maintien (3,3").

7. Le dispositif de maintien d'une tige (1,1',1",1"') selon une quelconque des revendications 1 à 5, l'élément de manchon (9,9') étant configuré pour être bloqué par friction dans ladite partie réceptrice (8,8") du corps de maintien (3,3").

8. Le dispositif de maintien d'une tige (1"') selon une quelconque des revendications précédentes, l'élément d'ancrage comprenant une vis à os (26"').

9. Le dispositif de maintien d'une tige (1"') selon la revendication 8, ladite vis à os (26"') étant connectée de façon pivotante au corps de maintien (3").

10. Le dispositif de maintien d'une tige (1,1') selon une quelconque des revendications précédentes, l'élément d'ancrage comprenant un élément allongé flexible (19).

11. Le dispositif de maintien d'une tige (1,1') selon la revendication 10, ledit élément allongé flexible (19) étant configuré pour être maintenu par friction entre l'élément de manchon (9,9') et la partie réceptrice (8,8') du corps de maintien (3) par l'engagement de l'élément de fermeture (4) avec la partie d'engagement (7,7") du corps de maintien (3).

12. Le dispositif de maintien d'une tige (1") selon une quelconque des revendications précédentes, ledit élément d'ancrage comprenant un crochet (25").

13. Le dispositif de maintien d'une tige (1") selon la revendication 12, le crochet (25") étant solidaire du corps de maintien (3 ").

14. Le dispositif de maintien d'une tige (1,1',1",1"') selon une quelconque des revendications précédentes, ledit élément de fermeture (4,4") comprenant un filet et la partie d'engagement (7,7") du corps de maintien (3,3") comprenant un filet complémentaire.

15. Le dispositif de maintien d'une tige (1,1') selon une quelconque des revendications précédentes, l'élément de fermeture (4) étant articulé sur le corps de maintien (3).
